(19) Europäisches Patentamt
European Patent Office
Office européen des brevets

(11) Veröffentlichungsnummer: **0 124 706**
**B1**

# (12) EUROPÄISCHE PATENTSCHRIFT

(45) Veröffentlichungstag der Patentschrift: **14.12.88**

(51) Int. Cl.⁴: **B 01 J 27/18,** C 07 C 51/25, C 07 C 57/04

(21) Anmeldenummer: **84102337.7**

(22) Anmeldetag: **05.03.84**

(54) **Verfahren und Katalysator zur Herstellung von Methacrylsäure.**

(30) Priorität: **11.03.83 DE 3308625**

(43) Veröffentlichungstag der Anmeldung: **14.11.84 Patentblatt 84/46**

(45) Bekanntmachung des Hinweises auf die Patenterteilung: **14.12.88 Patentblatt 88/50**

(84) Benannte Vertragsstaaten: **BE DE FR GB IT NL**

(56) Entgegenhaltungen:
**FR-A-2 305 424**
**US-A-4 042 533**
**US-A-4 070 397**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Duembgen, Gerd, Dr., Sudetenstrasse 4, D-6701 Dannstadt- Schauernheim (DE)**
Erfinder: **Fouquet, Gerd, Dr., Maxburgstrasse 2, D-6730 Neustadt (DE)**
Erfinder: **Krabetz, Richard, Dr., Unterer Waldweg 8, D-6719 Kirchheim (DE)**
Erfinder: **Merger, Franz, Dr., Max- Slevogt- Strasse 25, D-6710 Frankenthal (DE)**
Erfinder: **Nees, Friedbert, Dr., Fichtestrasse 4, D-7513 Stutensee (DE)**

**EP 0 124 706 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Es sind zahlreiche Oxidationskatalysatoren und deren Verwendung zur Herstellung von Methacrylsäure durch Gasphasenoxidation von Methacrolein vorgeschlagen worden. Diese befriedigen jedoch nicht oder nur zum Teil die Anforderungen des technischen Betriebes hinsichtlich geringer Nebenproduktbildung, insbesondere an Essigsäure, Malein- und Citraconsäure, bei technisch sinnvollen Teilchengrößen des Katalysators und hohen Raumgeschwindigkeiten des Methacroleins.

Aus der US-PS-3 772 381 sind z. B. Katalysatoren bekannt, die Mo, Cu, P, Sb und Cs und/oder Ca enthalten. Mit diesen Katalysatoren werden jedoch nur unbefriedigende Selektivitäten von 76 % für die Methacrylsäurebildung bei Methacroleinumsätzen von 75 % erreicht. In der DE-OS-2 523 757 werden Katalysatoren vorgeschlagen, die zusätzlich zu Mo, Cu und P wenigstens ein Alkalimetall und wenigstens ein Metall, ausgewählt aus der Gruppe Sb, V, W, Fe, Mn und Sn enthalten. Mit diesen Katalysatoren werden zwar im Dauerbetrieb Umsätze an Methacrolein bis 91,5 % und Selektivitäten von 82 % erzielt, die niedrigen Durchsätze (space velocity) von 1000 $h^{-1}$ und relativ hohen Temperaturen von 325°C und mehr befriedigen jedoch nicht bei technische Betrieb. Auch Oxidationskatalysatoren der aus der GB-PS-2 046 252 bekannten Art, die Mo, P, V sowie gegebenenfalls As und Cu oder andere kationische Elemente enthalten, zeigen zwar hohe katalytische Wirksamkeit, jedoch nur bei technisch uninteressanten Katalysatorteilchengrößen unter 2 mm und verhältnismäßig hohen Temperaturen von 330°C. Oxidationskatalysatoren, die in Gegenwart hoher Chlorionenkonzentrationen von etwa 1 bis 5 Äquivalenten je Äquivalent Molybdän hergestellt werden, wie beispielsweise die Mo, P und W enthaltenden Katalysatoren der EP-PS-0 010 429 oder die Mo, P, Sb und gegebenenfalls W enthaltenden Katalysatoren gemäß der US-PS-3 965 163 zeigen zwar eine verhältnismäßig gute katalytische Wirksamkeit bei kurzen Betriebszeiten, doch ist es schwierig, langlebige und ausreichend selektive Katalysatoren dieser Art reproduzierbar herzustellen. Außerdem neigen die genannten Katalysatoren zur verstärkten Bildung von Essigsäure, wenn sie in technisch sinnvollen Teilchengrößen von mindestens 3 mm und mehr eingesetzt werden. Aus der GB-PS-2 001 256 sind weitere oxidische Katalysatoren bekannt, die Mo, P, As, C, und Cr enthalten und die in Anwesenheit oder Abwesenheit einer dibasischen Carbonsäure, Oxycarbonsäure, Mannit oder Pyrogallol als Reduktionsmittel hergestellt sind. Aus der US-P-4 070 397 ist ein Verfahren zur Methacrolein-Oxidation zu Methacrylsäure bekannt, bei dem ein Katalysator de allgemeinen Formel $Sb_2W_aP_bV_cMo_{12}O_x$ mit Z = 0,1 bis 6, a und c = 0,1 bis 12 und b > 6 engesetzt wird, und die US-P-4 042 533 beschreibt einen Katalysator der allgemeinen Formel $Mo_aV_bW_cX_dY_eO_f$ in der X für Re und Ti, Y für u.a. Cu, P, Alkalimetalle, a für 6 bis 18, b für 0,1 bis 10, c für 0,1 bis 6, d für 0,01 bis 5 und e für 0 bis 5 stehen, wobei beide Katalysatoren im Grenzfall die Komponenten W und Mo in äquimolaren Mengen enthalten können. Die Eigenschaften dieser und der obengenannten Katalysatoren sind im allgemeinen jedoch dann unbefriedigend, wenn für die Herstellung von Methacrylsäure als Rohstoff Methacrolein eingesetzt wird, das durch Kondensation von Propanol mit Formaldehyd hergestellt ist. Dieses enthält als herstellungsbedingte Verunreinigungen neben nicht umgesetztem Propanol organische Amine, Dimere des Methacroleins und Methylpentenal. Bereits geringe Mengen dieser Verunreinigungen führen aber im allgemeinen zu einer Leistungsminderung derartiger Katalysatoren.

Es bestand daher ein technisches Interesse, Oxidationskatalysatoren, insbesondere für die Oxidation von Methacrolein zu Methacrylsäure in der Gasphase, zu finden, die bei Einsatz technischer Methacroleinqualitäten und technisch bei Festbettreaktionen üblicher Teilchengröße des Katalysators hohe Ausbeuten und eine geringe Bildung an Nebenprodukten auch bei hohen Raumbelastungen über lange Betriebszeiten gewährleisten.

Es wurde nun gefunden, daß diese Aufgabe mit Oxidationskatalysatoren der allgemeinen Formel

$W_6Mo_aV_bP_cCu_dAs_eSb_fX_gY_hO_x$

gelöst werden kann, in der

X für K, Rb und/oder Cs,
Y für Nb, Fe, Mn, Sn, Li, Na, Sr, Rh, Ce, Ti und/oder Cr
a für 2,0 bis < 6,
b für 0 bis 3,
c für 0,1 bis 3,
d für 0,01 bis 1,
e für 0 bis 1,
f für 0 bis 2,
g für 0,01 bis 3,
h für 0 bis 1,
x für die zur Absättigung der Valenzen der anderen Bestandteile formal erforderliche Anzahl der Sauerstoffatome steht.

Die gegebenenfalls vorhandenen $NH_4^+$-Ionen sind in die Formel aus formalen Gründen nicht mit aufgenommen. Die Katalysatoren können andere Komponenten in Mengen, die durch den natürlichen Gehalt der eingesetzten Rohstoffe gegeben sind, enthalten.

Hinsichtlich der Zusammensetzung werden solche Oxidationskatalysatoren der oben genannten Formel bevorzugt, in denen

a für 4,0 bis 5,9;
b für 0,1 bis 2;
c für 0,5 bis 1,5;
d für 0,05 bis 0,5;
e für 0,01 bis 0,5;
f für 0,01 bis 1,5;
g für 0,05 bis 1,5;
h für 0 bis 0,5

stehen. Von den Komponenten der Gruppe Y wird Ti, Sn und/oder Rh gegebenenfalls in Kombination mit einer weiteren Komponente dieser Gruppe, vorgezogen.

Die Katalysatoren werden im allgemeinen so hergestellt, daß Verbindungen der Einzelkomponenten in wäßrigem Medium, d.h. im wäßriger Lösung oder Suspension, unter Bedingungen, die zur Bildung der Phosphor-haltigen Heteropolysäuren des Molybdäns und Wolframs oder ihrer Salze führen, vereinigt, anschließend getrocknet und verformt und schließlich vorteilhaft bei höheren Temperaturen durch Calcinieren aktiviert werden.

Geeignet als Molybdän- und Wolframquelle sind beispielsweise Molybdänsäure, Ammoniummolybdat, Phosphomolybdänsäure und ihr Ammoniumsalz, Wolframsäure, Ammoniumwolframat, Phosphowolframsäure und ihr Ammoniumsalz. Obwohl auch andere Verbindungen eingesetzt werden können, werden die genannten Verbindungen bevorzugt, insbesondere der Einsatz der Phosphowolframsäure als Wolfram- und der Einsatz von Ammoniummolybdat, Molybdänsäure und Phosphomolybdänsäure als Molybdänquelle. Arsen wird vorteilhaft als Oxid bzw. Säure oder als Ammoniumsalz der Säuren eingesetzt. Als Phorphorquelle können neben den obengenannten Heteropolysäuren verschiedene Verbindungen eingesetzt werden, bevorzugt werden jedoch die Phosphorsäure und ihre Ammoniumsalze. Die Elemente der Gruppen X und Y sowie Cu und Sb können beispielsweise in Form der Oxide, Carbonate, Nitrate, Chloride, Fluoride, Formiate, Oxalate oder Acetate eingesetzt werden. Bevorzugt wird jedoch der Einsatz in Form der Salze niedermolekularer Mono- und Dicarbonsäuren. Die Anwesenheit reduzierend wirkender organischer Stoffe, insbesondere von niedermolekularen Monocarbonsäuren, wie Ameisensäure, Essigsäure, Di- und Oxycarbonsäuren wie Oxalsäure, Weinsäure, Zitronensäure oder ihrer Salze, vorzugsweise Ameisensäure allein oder in Kombination mit den oben genannten Säuren, insbesondere Essigsäure, in der Ausgangslösung und während der Herstellung ist im allgemeinen vorteilhaft. Die Carbonsäuren können in Mengen von 0,02 bis 2 Mol, vorzugsweise 0,05 bis 1,5 Mol je Mol Wolfram zugesetzt werden. Höhere Chlorionenkonzentrationen können dagegen in manchen Fällen schädigend auf die Katalysatoren wirken. Die Chlorionenmenge in der Ausgangslösung soll daher vorzugsweise unter 0,3 insbesondere unter 0,25 Mol je Mol Molybdän liegen.

Die Vereinigung der Komponenten kann bei Raumtemperatur oder auch erhöhter Temperatur durchgeführt werden. Man kann beispielsweise die wäßrigen Lösungen oder Suspensionen der Molybdänsäure, Phosphorsäure, Arsensäure, des Antimon(III)oxids und Kupferoxids oder Kupfersalzes mischen und anschließemd beispielsweise 2 bis 24 Stunden unter Rückfluß zum Sieden erhitzen. Bei einer anderen Ausführungsform werden die wäßrigen Lösungen wasserlöslicher Verbindungen der Komponenten, beispielsweise Ammoniumheptamolybdat, Di-ammoniumphosphat, Di-ammoniumarsenat oder Arsensäure und Antimontrichlorid, in salz- oder vorzugsweise in ameisen-, wein-, bernstein- oder zitronensaurer Lösung bei Raumtemperatur gemischt, mit der wäßrigen Lösung von z. B. der Phosphorwolframsäure vereinigt und nach Zugabe der kationischen Zusatzkomponenten bei erhöhter Temperatur entwässert.

Die Entwässerung bzw. Trocknung der wäßrigen Suspension der Komponenten geschieht im allgemeinen durch Eindampfen in Rührkesseln bei Temperaturen unter 140°C oder durch Sprühtrocknung bei Austrittstemperaturen zwischen 80 und 140°C.

Nach dem Trocknen werden die erhaltenen Massen meist auf Teilchengrößen von 200 bis 1200 µm gemahlen und gegebenenfalls nach Zusatz üblicher Träger, wie $SiO_2$ oder Aluminiumoxiden sowie gegebenenfalls von Gleitmitteln, wie Graphit, zu Kugeln, Tabletten, Ringen oder anderen Formen verpreßt. Man kann dann anschließend in der Luft, unter Stickstoff oder schwach reduzierender Atmosphäre bei geringen Gasströmungen und Temperaturen von 180 bis 400°C, vorzugsweise von 220 bis 380°C, insbesondere von 320 bis 360°C, calcinieren bzw. aktivieren. Die Trägerstoffe können auch während des Eindampfens der Katalysatorsuspension zugegeben werden, wodurch die Katalysatorkomponenten auf den Trägern abgeschieden werden. Schließlich können auch die getrockneten und gemahlenen Katalysatormassen ohne Zusatz von Trägern bei den genannten Temperaturen calciniert und anschließend zu geformten Gebilden verarbeitet oder auf Träger, insbesondere auf kugelförmige Träger, in Form von Schalen in an sich üblicher Weise, z. B. nach den aus den US-PSen-4 305 843 und 4 297 247 bekannten Verfahren, aufgebracht werden. Die katalytisch aktiven Massen weisen nach dem Calcinieren ausschließlich die Struktur einer gestörten Heteropolysäure oder ihrer Salze mit charakteristischen Röntgenbeugungslinien auf. Sie sind besonders für die Oxidation von Methacrolein zu Methacrylsäure unter an sich üblichen Bedingungen in der Gasphase geeignet, insbesondere dann, wenn als Ausgangsmaterial Methacrolein verwendet wird, das durch Kondensation von Formaldehyd mit Propionaldehyd hergestellt ist.

Bei der Gasphasenoxidation von Methacrolein werden als Oxidationsmittel Sauerstoff und Wasserdampf

enthaltende Gasgemische eingesetzt, die über im allgemeinen fest in Katalysatorbetten angeordnete Katalysatoren geleitet werden. Man arbeitet dabei im allgemeinen bei Drücken von 1 bis 5 bar, vorteilhaft von 1 bis 2,5 bar. Im allgemeinen beträgt bei dem Verfahren die auf Standardbedingungen bezogene Verweilzeit der Methacrolein enthaltenden Gasgemische 0,5 bis 5 sec. und Verweilzeiten von 1 bis 4 sec. bei Temperaturen im Bereich von 200 bis 340°C, insbesondere bei 220 bis 320°C, werden vorgezogen. Zusätzlich zu Sauerstoff, Methacrolein und Wasserdampf enthalten die Reaktionsgase im allgemeinen indifferente Gase, insbesondere Stickstoff, und der Sauerstoff wird im allgemeinen als Luft zugeführt. Er kann jedoch auch als Reinsauerstoff eingesetzt werden. Zudem enthält das Reaktionsgas im allgemeinen Kohlenoxide, insbesondere dann, wenn nach der Abtrennung der entstandenen Methacrylsäure restliches Reaktionsabgas als Verdünnungsmittel zusammen mit nichtumgesetztem Methacrolein in die Oxidationsreaktion zurückgeführt wird. Im Reaktionsgas beträgt das molare Verhältnis von Methacrolein: Sauerstoff: Wasser: Inertgas meist 1 : (1 bis 6) : (1 bis 20) : (4 bis 50), vorzugsweise 1 : (1,5 bis 4) : (2 bis 10) : (6 bis 30). Die Abtrennung der Methacrylsäure von den heißen Reaktionsabgasen kann in an sich üblicher Weise, meist durch Abschrecken mit Wasser, durchgeführt werden.

Das Methacrolein kann nach verschiedenen Verfahren gewonnen sein, z. B. durch Gasphasenoxidation von tert.-Butylalkohol, Isobutylen oder $C_4$-Gemischen oder durch Kondensation von Propionaldehyd mit Formaldehyd. Der Einsatz der erfindungsgemäßen Katalysatoren ist von besonderem Vorteil, wenn Methacrolein eingesetzt wird, das durch Kondensation von Propionaldehyd mit Formaldehyd in Gegenwart von Salzen sekundärer Amine oder mit Aminalen in Gegenwart von Säuren in wäßriger Lösung hergestellt ist. Technische Qualitäten, die auf diese Weise hergestellt sind, haben im allgemeinen einen Reinheitsgrad von 94 bis 99 % und enthalten neben nicht umgesetztem Propionaldehyd geringe Mengen organische Amine, wie Diethylamin oder Diethanolamin, Methylpentenal und Dimere des Methacroleins. Die Reinheitsangaben beziehen sich auf wasserfreies Rohmethacrolein, das bis zu 3,5 Gew.-% Wasser enthalten kann. Soweit nicht umgesetztes Methacrolein und nicht kondensierte Reaktionsabgase in die Oxidationsreaktion zurückgeführt werden, kann das Synthesegasgemisch auch geringe Mengen leicht flüchtiger Nebenprodukte, wie Kohlenoxide oder Acrolein, enthalten.

Bei der technischen Durchführung des Verfahrens arbeitet man meist in sogenannten Rohrbündelreaktoren, in denen die Katalysatoren fest angeordnet sind. Zur Vermeidung von örtlichen Überhitzungen kann die Katalysatoraktivität derart verändert werden, daß sie im Reaktionsrohr in Strömungsrichtung der Reaktionsgase stetig oder in Stufen ansteigt. Dies kann z. B. durch Verdünnen des Katalysators mit weniger aktiven oder inaktiven Katalysator- oder Trägerformlingen oder durch Einsatz von 2 oder mehr Katalysatoren mit unterschiedlicher Aktivität und/oder Selektivität erfolgen. Es ist auch möglich, die erfindungsgemäße Oxidation des Methacroleins zu Methacrylsäure in der Wirbelschicht durchzuführen, fest angeordnete Katalysatorschichten werden jedoch vorgezogen.

Bei der Aufarbeitung der Reaktionsgase, bei der vor dem Waschen, z. B. mit Wasser, auch indirekt gekühlt werden kann, werden meist wäßrige Lösungen der Methacrylsäure erhalten, die gegebenenfalls zusätzlich geringe Mengen Essigsäure, Maleinsäure und Acrylsäure enthalten. Aus den erhaltenen, z. B. wäßrigen Lösungen der Methacrylsäure kann diese mit geeigneten Lösungsmitteln, wie beispielsweise Methylmethacrylat, in an sich üblicher Weise extrahiert und entweder direkt mit Alkanolen, wie besonders Methanol, verestert oder durch Destillation aus dem Extrakt abdestilliert und von Nebenprodukten getrennt werden. Das nicht umgesetzte Methacrolein kamn aus dem wäßrigen Kondensat destilliert oder z. B. mit Wasserdampf ausgetrieben und der Oxidationsreaktion wieder zugeführt werden.

Die erfindungsgemäßen Katalysatoren zeigen auch bei anderen Oxidationsreaktionen, z. B. bei der Oxidation von Acrolein zu Acrylsäure oder bei der Oxidation von substituierten Toluolderivaten zu substituierten Benzaldehyden und Benzoesäuren gute Wirksamkeit und Selektivität.

In den folgenden Beispielen wird Methacrolein eines Reinheitsgrades von 97 bis 99 % eingesetzt, daß neben Wasser und Propionaldehyd geringe Mengen an sekundären Amimen und Nebenprodukten der Methacroleinsynthese aus Propionaldehyd und Formaldehyd enthält. Die darin angegebenen Teile und Prozente beziehen sich, soweit nicht anders angegeben, auf das Gewicht. Die darin angegebenen Volumenteile verhalten sich zu den Gewichtsteilen wie das Liter zum Kilogramm.

**Beispiel 1**

Es wurde ein Katalysator der folgenden Zusammensetzung hergestellt:

$W_6Mo_{5,5}V_{0,3}P_1As_{0,2}Sb_{0,2}Cu_{0,25}K_1O_x$

80 Volumenteile Katalysatortabletten werden in ein salzbadbeheiztes Reaktionsrohr von 16 mm Durchmesser eingefüllt. Über den Katalysator wird mit einer Raumgeschwindigkeit von 1320 $h^{-1}$ eine Gasmischung aus 3,3 Vol.-% Methacrolein, 9,1 Vol.-% Sauerstoff, 29,5 Vol.-% Wasserdampf und 58,1 Vol.-% Stickstoff geleitet. Bei einer Badtemperatur von 310°C beträgt der Umsatz 85,3 Mol.-%, die Methacrylsäure-Selektivität 88 Mol.-% und die Essigsäure-Selektivität 2,6 Mol.-%. Malein- und Citraconsäure werden in einer Gesamtmenge von 0,8 %, bezogen auf die Menge an Methacrylsäure, gebildet.

**Vergleichsversuche**

1A) Nach Beispiel 1 wird ein Katalysator hergestellt mit der Änderung, daß der Zusatz von Kaliumnitrat unterlassen wird. Unter den Testbedingungen von Beispiel 1 wird bei einer Badtemperatur von 310°C ein Umsatz von 69,9 Mol.-%, eine Methacrylsäure-Selektivität von 82,5 Mol.-% und eine Essigsäure-Selektivität von 3,7 Mol.-% gemessen (Malein- und Citraconsäure zusammen 1,2 %).

1B) In einem weiteren Vergleichsversuch wird nach Beispiel CE 16 der Deutschen Offenlegungsschrift 30 10 434 ein Katalysator der Zusammensetzung $Mo_{10}V_1P_1As_{0,2}Cu_{0,25}K_{0,1}O_x$ hergestellt und, nach Verformung zu 3 x 3 mm Tabletten, wie in Beispiel 1 beschrieben, unter den Testbedingungen von Beispiel 1 geprüft. Bei einer Badtemperatur von 324°C wird ein Umsatz von 85,2 Mol.-% und eine Methacrylsäure-Selektivität von 74,4 Mol.-% erhalten. Die Essigsäure-Selektivität beträgt 8,6 Mol.-% (Malein- und Citraconsäure zusammen 2,2 %).

1C) Nach den Angaben von Beispiel 1 wird ein Katalysator hergestellt, in dem aber Mo vollständig durch die gleiche molare Menge W ersetzt ist (Zusammensetzung: $W_{11,5}V_{0,3}P_1As_{0,2}Sb_{0,2}Cu_{0,25}K_1O_x$). Unter den Testbedingungen von Beispiel 1 wird ein Umsatz von 53,6 Mol.-%, eine Methacrylsäure-Selektivität von 67,2 Mol.-% und eine Essigsäure-Selektivität von 12,2 Mol.-% bei einem Malein- und Citraconsäure-Anfall von zusammen 1,4 % erreicht.

**Beispiel 2**

Beispiel 1 wird wiederholt mit der Änderung, daß über 80 Volumenteile Katalysator eine Gasmischung aus 6,3 Vol.-% Methacrolein, 11,8 Vol.-% Sauerstoff, 22 Vol.-% Wasserdampf und 59,9 Vol.-% Stickstoff bei einer Raumbelastung von 1100 $h^{-1}$ geleitet wird. Bei einer Badtemperatur von 294°C beträgt der Umsatz 62 Mol.-%, die Methacrylsäure-Selektivität 90,1 Mol.-% und die Essigsäure-Selektivität 1,9 Mol.-% Malein- und Citraconsäure treten in einer Menge von 0,9 %, bezogen auf Methacrylsäure, auf.

Nach 90 Betriebstagen wird bei der gleichen Badtemperatur ein Umsatz von 59,5 Mol.-%, eine Methacrylsäure-Selektivität von 90,8 Mol.-% und eine Essigsäure-Selektivität von 1,1 Mol.-% erhalten (Malein- und Citraconsäure zusammen 0,82 %).

**Beispiel 3**

Nach der Herstellungsvorschrift des Beispiels 1 wird ein Katalysator der Zusammensetzung $W_6Mo_{5,8}P_1As_{0,2}Sb_{0,2}Cu_{0,25}K_{0,2}O_x$ hergestellt und unter den Testbedingumgen des Beispiels 1 geprüft. Bei einer Badtemperatur von 294°C wird ein Umsatz von 85,6 Mol.-%, eine Methacrylsäure-Selektivität von 85,7 Mol.-% und eine Essigsäure-Selektivität von 3,0 Mol.-% erhalten.

**Vergleichsversuche**

3A) Nach der allgemeinen Herstellvorschrift von Beispiel 3 wird ein Katalysator ohne Zusatz von Kaliumnitrat und mit erhöhter Molybdänmenge entsprechend der Formel $W_6Mo_{6,4}P_1As_{0,2}Cu_{0,25}O_x$ hergestellt. Unter den Testbedingungen des Beispiels 3 beträgt bei einer Badtemperatur von 306°C der Umsatz 67,2 Mol.-%, die Methacrylsäure-Selektivität 81,7 Mol.-% und die Essigsäure-Selektivität 5,1 Mol.-% (Malein- und Citraconsäure zusammen 1,8 %).

3B) Mit einem Katalysator der Zusammensetzung $W_2Mo_{10}P_1As_{0,2}Sb_{0,2}Cu_{0,25}O_x$ wird bei einer Badtemperatur von 294°C ein Umsatz von 86,4 Mol.-%, eine Methacrylsäure-Selektivität von 80,9 Mol.-% und eine Essigsäure-Selektivität von 5,2 Mol.-% gemessen.

**Beispiel 4**

Nach den Amgaben von Beispiel 1 wird ein Katalysator der allgemeinen Formel $W_6Mo_{5,8}V_{0,6}P_1As_{0,2}Sb_{0,2}Cu_{0,25}K_1O_x$ hergestellt. 80 Volumenteile der calcinierten Katalysatortabletten werden unter den in Beispiel 2 angegebenen Bedingungen bei einer Badtemperatur von 316°C getestet. Der Umsatz beträgt 63,5 Mol.-%, die Essigsäure-Selektivität 2,7 Mol.-% und die Methacrylsäure-Selektivität 90,3 Mol.-% (Malein- und Citraconsäure zusammen 0,62 %).

**Beispiele 5 bis 14**

Entsprechend der Herstellungsvorschrift des Beispiels 1 werden weitere Katalysatoren hergestellt, mit der Änderung, daß weitere Zusatzkomponenten eingeführt oder die Mengenverhältnisse der Komponenten geändert werden. Die Katalysatoren werden unter den Testbedingungen von Beispiel 2 geprüft. Die Katalysatorzusammensetzungen sind in der folgenden Tabelle zusammengefaßt. Die Zusatzkomponenten werden in folgender Form eingeführt: Mangan(II)acetat-4-hydrat, Eisen(II)oxalat, Niobpentoxid, Ammoniumchromat, Anatas, Zinn(II)oxid, Rubidiumnitrat, Caesiumnitrat, Lithiumhydroxid, Natriumhydroxid, Rhodium(III)chlorid-hydrat, Cernitrat. Die Katalysatoren sind praktisch gleich wirksam wie der Katalysator von Beispiel 1. Die jeweiligen Umsätze und Methacrylsäure-Selektivitäten jeweils in Mol.-% und die Badtemperaturen sind in der folgenden Tabelle zusammengestellt. Die Essigsäure-Selektivitäten liegen jeweils unter 3 Mol.-%, die Menge an Malein- und Citraconsäure zusammen unter 0,7 %, bezogen auf die Methacrylsäuremenge.

**Tabelle**

| Beispiel | Katalysator | Badtemperatur °C | Umsatz | Selektivität |
|---|---|---|---|---|
| 5 | $W_6Mo_{5,8}V_{0,3}P_1As_{0,2}Sb_{0,2}Cu_{0,25}Cs_1Na_{0,01}$ | 294 | 56 | 90,1 |
| 6 | $W_6Mo_{5,8}V_{0,3}P_1As_{0,2}Sb_{0,2}Cu_{0,25}Rb_{0,5}Li_{0,01}$ | 294 | 61 | 88,5 |
| 7 | $W_6Mo_{5,8}V_{0,3}P_1As_{0,2}Sb_{0,2}Cu_{0,25}K_1Ti_{0,2}$ | 287 | 61,7 | 88, |
| 8 | $W_6Mo_{5,8}V_{0,3}P_1As_{0,2}Sb_{0,2}Cu_{0,25}K_1Rh_{0,1}$ | 286 | 61,5 | 86,1 |
| 9 | $W_6Mo_{5,8}V_{0,3}P_1As_{0,2}Sb_{1,0}Cu_{0,25}K_1Ce_{0,05}$ | 292 | 59,4 | 88,0 |
| 10 | $W_6Mo_{5,8}V_{0,3}P_{1,1}As_{0,3}Sb_{0,2}Cu_{0,25}Sr_{0,1}K_{0,8}$ | 288 | 62,0 | 90,5 |
| 11 | $W_6Mo_{5,8}V_{0,3}P_1As_{0,2}Sb_{0,2}Cu_{0,25}K_1Fe_{0,05}$ | 294 | 59,3 | 87,0 |
| 12 | $W_6Mo_{5,8}V_{0,3}P_1As_{0,2}Sb_{0,2}Cu_{0,2}K_{0,2}Mn_{0,05}$ | 294 | 55 | 89,3 |
| 13 | $W_6Mo_{5,8}V_{0,3}P_1As_{0,2}Sb_{0,2}Cu_{0,25}K_{1,1}Sn_{0,05}$ | 292 | 63,1 | 87,2 |
| 14 | $W_6Mo_{5,8}V_{0,3}P_1As_{0,2}Sb_{0,2}Cu_{0,2}Cr_{0,05}K_{0,2}Nb_{0,2}$ | 305 | 58 | 88,2 |

**Beispiel 15**

Nach den Angaben in Beispiel 1 wird ein Katalysator der allgemeinen Formel $W_6Mo_4V_{0,3}P_1As_{0,3}Sb_{0,25}Cu_{0,3}Cs_{0,8}O_x$ hergestellt und unter den Bedingungen des Beispiels 2 getestet. Bei einer Badtemperatur von 324°C beträgt der Umsatz 54,5 Mol.-%, die Methacrylsäure-Selektivität 85,5 Mol.-%, die Essigsäure-Selektivität 2,7 Mol.-% und der Anfall an Malein- und Citraconsäure zusammen 0,85 %, bezogen auf die Methacrylsäure.

**Patentansprüche**

1. Oxidationskatalysator der allgemeinen Formel
$W_6Mo_aV_bP_cCu_dAs_eSb_fX_gY_hO_x$
in der
X für K, Rb und/oder Cs,
Y für Nb, Fe, Mn, Sn, Li, Na, Sr, Rh, Ce, Ti und/ oder Cr
a für 2,0 bis 6,
b für 0 bis 3,
c für 0,1 bis 3,
d für 0,01 bis 1,
e für 0 bis 1,
f für 0 bis 2,
g für 0,01 bis 3,
h für 0 bis 1,
x für die zur Absättigung der Valenzen der Katalysatorkomponenten formal erforderliche Anzahl der Sauerstoffatome stehen.

2. Oxidationskatalysator nach Anspruch 1, dadurch gekennzeichnet, daß a für 4,0 bis 5,9, b für 0,1 bis 2, c für 0,5 bis 1,5, d für 0,05 bis 0,5, e für 0,01 bis 0,5, f für 0,01 bis 1,5, g für 0,05 bis 1,5, h für 0 bis 0,5 steht.

3. Verwendung von oxidationskatalysatoren gemäß den Ansprüchen 1 bis 2 bei der Herstellung von Methacrylsäure durch Oxidation von Methacrolein in der Gasphase mit Sauerstoff und Wasserdampf enthaltenden Gasgemischen.

4. Verfahren zur Herstellung von Methacrylsäure durch Oxidation von Methacrolein mit Sauerstoff und Wasserdampf enthaltenden Gasgemischen bei Temperaturen von 200 bis 340°C an Katalysatoren, die Molybdän, Wolfram, Antinon und Phosphor enthalten, und Abtrennen der Methacrylsäure aus den Reaktionsgasen in an sich üblicher Weise, dadurch gekennzeichnet, daß man die Oxidation an einem Katalysator gemäß Anspruch 1 durchführt.

**Claims**

1. An oxidation catalyst of the formula
$W_6Mo_aV_bP_cCu_dAs_eSb_fX_gY_hO_x$
where
X is K, Rb and/or Cs,
Y is Nb, Fe, Mn, Sn, Li, Na, Sr, Rh, Ce, Ti and/or Cr,
a is from 2,0 to 6,
b is from 0 to 3,
c is from 0,1 to 3,
d is from 0,01 to 1,
e is from 0 to 1,
f is from 0 to 2,
g is from 0,01 to 3,
h is from 0 to 1 and
x is the number of oxygen atoms formally required to saturate the valencies of the catalyst components.

2. An oxidation catalyst as claimed in claim 1, wherein a is from 4.0 to 5.9, b is from 0.1 to 2, c is from 0.5 to 1.5, d is from 0.05 to 0.5, e is from 0.01 to 0.5, f is from 0.01 to 1.5, g is from 0.05 to 1.5 and h is from 0 to 0.5.

3. Use of an oxidation catalyst as claimed in either of claims 1 or 2 in the preparation of methacrylic acid by oxidation of methacrolein in the gaseous phase with a gas mixture containing oxygen and steam.

4. A process for the preparation of methacrylic acid by oxidation of methacrolein with a gas mixture containing oxygen and steam, at from 200 to 340°C, over a catalyst which contains molybdenum, tungsten, antimony and phosphorus, and isolation of the methacrylic acid from the reaction gases in a conventional manner, wherein the oxidation is carried out over a catalyst as claimed in claim 1.

**Revendications**

1. Catalyseur d'oxydation de formule genérale
$W_6Mo_aV_bP_cCu_dAs_eSb_fX_gY_hO_x$
dans laquelle
X est mis pour K, Rb et/ou Cs,
Y pour Nb, Fe, Mn, Sn, Li, Na, Sr, Rh, Ce, Ti et/ou Cr,
a pour 2,0 à 6,
b pour 0 à 3,
c pour 0,1 à 3,
d pour 0,01 à 1,
e pour 0 à 1,
f pour 0 à 2,
g pour 0,01 à 3,
h pour 0 à 1,
x pour le nombre des atomes d'oxygène nécessaire, de par la formule, pour la saturation des valences des composants du catalyseur.

2. Catalyseur d'oxydation selon la revendication 1, caractérisé en ce que a est mis pour 4,0 à 5,9, b pour 0,1 à 2, c pour 0,5 à 1,5, d pour 0,05 à 0,5, e pour 0,01 à 0,5, f pour 0,01 à 1,5, g pour 0,05 à 1,5 et h pour 0 à 0,5.

3. Utilisation de catalyseurs d'oxydation selon la revendication 1 ou 2 dans la préparation d'acide méthacrylique par oxydation de méthacroléine en phase gazeuse par des mélanges de gaz contenant de l'oxygène et de la vapeur d'eau.

4. Procéde de préparation d'acide methacrylique par oxydation de méthacroléine par des mélanges de gaz contenant de l'oxygène et de la vapeur d'eau, à des temperatures de 200 à 340°C et en présence de catalyseurs qui contiennent du molybdène, du tungstene, de l'antimoine et du phosphore, et par séparation de l'acide methacrylique d'avec les gaz de réaction de façon en soi connue, caractérisé en ce qu'on mène l'oxydation en présence d'un catalyseur selon la revendication 1.